# EUROPÄISCHE PATENTSCHRIFT

(11) **EP 2 467 078 B1**
(45) Veröffentlichungstag und Bekanntmachung des Hinweises auf die Patenterteilung: **07.11.2018**
(21) Anmeldenummer: 10751797.1
(22) Anmeldetag: 11.08.2010
(51) Int. Cl.: A61B 18/14

(54) **ELEKTROCHIRURGISCHE ZANGE**
ELECTROSURGICAL FORCEPS
PINCE ÉLECTROCHIRURGICALE

(30) Priorität: 20.08.2009 DE 102009038171; 14.10.2009 DE 102009049401
(43) Veröffentlichungstag der Anmeldung: 27.06.2012
(73) Patentinhaber: Erbe Elektromedizin GmbH, 72072 Tübingen (DE)
(72) Erfinder: SCHÄLLER, Daniel, 72070 Tübingen (DE); FISCHER, Klaus, 72202 Nagold (DE); HAFNER, Dieter, 72072 Tübingen (DE)
(74) Vertreter: Bohnenberger, Johannes
(86) Internationale Anmeldenummer: PCT/EP2010/004924
(87) Internationale Veröffentlichungsnummer: WO 2011/020578

(56) Entgegenhaltungen:
- WO-A2-02/058542
- US-A1- 2004 049 185
- US-A1- 2005 159 745
- US-A1- 2005 171 535

## Beschreibung

Die Erfindung betrifft eine elektrochirurgische Zange, wie aus dem nächstliegenden Stand der Technik US2005171535 bekannt und im Oberbegriff des Anspruchs 1.

Elektrochirurgische Instrumente werden seit vielen Jahren in der Hochfrequenzchirurgie (HF-Chirurgie) eingesetzt, um biologisches Gewebe zu koagulieren oder auch zu schneiden. Bei einer Koagulation wird ein hochfrequenter Strom durch das zu behandelnde Gewebe geleitet, so dass sich dieses aufgrund von Eiweißgerinnung und Dehydration verändert. Das Gewebe zieht sich dabei derart zusammen, dass die Gefäße verschlossen und Blutungen gestillt werden. Nach der erfolgreichen Koagulation ist das Gewebe unter Vermeidung starker Blutungen vollständig durchtrennbar.

Aus der DE 44 218 22 ist ein entsprechendes endoskopisches, bipolares HF-Koagulationsgerät mit integrierter Schneideinrichtung bekannt. Hierbei handelt es sich um eine endoskopische Zange, die aus zwei gelenkig aneinander gelagerten Branchen besteht. Die Branchen weisen Maulteile auf, um Gewebe zu fassen. Jedes der Maulteile hat jeweils ein Paar von Elektroden, die sich wechselseitig entlang der Längsachse der Maulteile erstrecken. Im geschlossenen Zustand liegen die Paare von Elektroden einander gegenüber. Es ist möglich, Gewebe mittels der Maulteile zu fassen und dann einen HF-Strom zu applizieren, so dass das Gewebe zwischen den Elektrodenpaaren koaguliert wird. Die DE 44 218 22 hat eine mechanische Schneidvorrichtung, die mittig zwischen den Elektrodenpaaren angeordnet ist. Die Schneidvorrichtung umfasst ein Messer, das sich nach dem Fassen und Koagulieren des Gewebes entlang der Längsachse der Maulteile verschieben lässt. Somit dient dieses Messer zum Durchtrennen des koagulierten Gewebes.

Die mittels dieses HF-Koagulationsgeräts erzeugten Schnitte sind nicht stets befriedigend. Des Weiteren lässt sich die Schneidvorrichtung schwer bedienen.

Ausgehend von der DE 44 218 22 ist es Aufgabe der vorliegenden Erfindung, eine verbesserte elektrochirurgische Zange bereitzustellen.

Diese Aufgabe wird durch ein Instrument gemäß dem vorliegenden Anspruch 1 gelöst.

Ein Gedanke der vorliegenden Erfindung besteht also darin, an Stelle der mechanischen Schneidvorrichtung eine elektrochirurgische Schneidvorrichtung vorzusehen. Hierfür dient die Schneidelektrode, die unter Verwendung der Neutralelektrode einen zweiten HF-Strom appliziert, der derart ausgelegt ist, dass das gefasste Gewebe bzw. das gefasste Hohlorgan durchtrennt wird. Dieser Ansatz ist problematisch, da es bei der vorhergehenden Koagulation mittels des ersten HF-Stroms zu einer Austrocknung des Gewebes kommt. Um einen sauberen Schnitt mittels des zweiten HF-Stroms zu gewährleisten, schlägt die vorliegende Erfindung vor, die Koagulationselektroden derart auszubilden und auszurichten, dass im Schnittbereich - also der Bereich des Hohlorgans, der durchtrennt werden soll - keine Koagulation vorkommt. Es soll also vermieden werden, dass das Gewebe im Schnittbereich austrocknet, so dass beim Schneidvorgang ein ausreichend erhaltenes Gewebe vorliegt, das sich rasch und effizient mittels des zweiten HF-Stroms durchtrennen lässt.

Vorzugsweise werden die Koagulationselektroden derart weit voneinander beabstandet angeordnet, dass kein HF-Strom während einer Koagulationsphase - also während des zumindest teilweisen Verschließens des Hohlorgans - den Schnittbereich durchfließt. Zumindest sollten die Koagulationselektroden derart weit voneinander beabstandet angeordnet werden, dass der Stromfluss auch über eine längere Zeit in dem Schnittbereich so gering gehalten wird, dass es zu keiner, insbesondere für einen Schnitt, nachteiligen Gewebeveränderung kommt.

Die elektrochirurgische Zange kann mindestens eine Aussparung zur Aufnahme des Schnittbereichs beim Fassen des Hohlorgans aufweisen. Die Aussparung sorgt dafür, dass der Schnittbereich beim Fassen des Gewebes nicht oder nur in geringem Maße geschädigt wird. Somit soll erfindungsgemäß eine mechanische Schädigung des Schnittbereichs vor dem endgültigen Durchtrennen vermieden werden. Auch die mechanische Schädigung des Gewebes kann bei einem elektrochirurgischen Trennvorgang stören.

Die elektrochirurgische Zange kann mindestens eine erste und eine zweite zumindest abschnittsweise voneinander isolierte Neutralelektrode umfassen, die jeweils korrespondierend zu der ersten und der zweiten Koagulationselektrode angeordnet sind. Somit ist es möglich, zu jeder Koagulationselektrode eine entsprechend korrespondierende Neutralelektrode bereitzustellen. Durch dieses paarweise Anordnen der ersten Neutralelektrode und der ersten Koagulationselektrode bzw. der zweiten Neutralelektrode und der zweiten Koagulationselektrode ist es möglich, den Strompfad des ersten HF-Stroms derart zu führen, dass eine Schädigung des Gewebes im Schnittbereich vermieden wird.

Vorzugsweise werden die Neutralelektroden unmittelbar benachbart zu den Koagulationselektroden angeordnet. Das heißt im geschlossenen Zustand der Zange sind die Elektroden einander gegenüberliegend angeordnet.

Die erste und die zweite Neutralelektrode können im Wesentlichen flächig ausgebildet sein und eine Ebene aufspannen. Vorzugsweise sind die Neutralelektroden derart angeordnet, dass die Ebenen sich unter einem Winkel, insbesondere einem spitzen Winkel, schneiden. Der Winkel, unter dem sich die Ebenen der Neutralelektroden schneiden, sollte kleiner als 150 Grad, insbesondere kleiner als 130 Grad, insbesondere kleiner als 110 Grad, insbesondere kleiner als oder gleich 80 Grad sein. Durch das verkippte Anordnen der Neutralelektrode gegenüber der Fixierebene des Hohlorganes, wirkt die Neutralelektrode wie eine sehr geringflächige Elektrode. Bei der Koagulation kommt es zu einem hohen Stromeintrag, bei dem ein räumlich stark begrenzter Bereich des Organs koaguliert wird. Für den Schneidvorgang mittels der Schneidvorrichtung ist dies vorteilhaft.

Ebenso können die Koagulationselektroden derart flächig ausgebildet sein, dass auch diese eine Ebene aufspannen. Auch die Koagulationselektroden sollten derart angeordnet sein, dass sich die Ebenen der Koagulationselektroden unter einem Winkel schneiden. Auch dieser Schnittwinkel sollte kleiner als 150 Grad, insbesondere kleiner als 130 Grad, insbesondere kleiner als 110 Grad, insbesondere kleiner als oder gleich 80 Grad sein. In einem Ausführungsbeispiel kann dieser Winkel ein spitzer Winkel sein. Somit können auch die Koagulationselektroden derart angeordnet werden, dass die Wirkfläche der Elektroden wesentlich geringer ist als deren tatsächliche Fläche. Dies ermöglicht eine schnelle und effiziente Koagulation. Vorzugsweise ist die erste Neutralelektrode parallel zur ersten Koagulationselektrode und die zweite Neutralelektrode parallel zur zweiten Neutralelektrode angeordnet, so dass das Gewebe optimal koaguliert wird. Durch ein Verkippen des jeweiligen Elektrodenpaars kommt es zu einem besonders vorteilhaften Koagulationsvorgang beim Schließen der Branchen und somit beim Fassen des Gewebes.

Die Koagulationselektroden können eine geringe Koagulationselektrodenbreite, insbesondere von kleiner gleich 3 mm oder kleiner gleich 2 mm haben. Vorzugsweise erstrecken sich die Koagulationselektroden entlang der Längsachse der elektrochirurgischen Zange, wobei deren Breite im Vergleich zu deren Länge relativ gering ist. Die durch derart schmal ausgeprägte Elektroden applizierte HF-Energie konzentriert sich auf einen relativ schmalen Abschnitt des Hohlorgans. Somit kann eine erfolgreiche Koagulation bereits nach einer sehr geringen Applikationszeit erzielt werden, wobei sich der Koagulationssaum minimal ausbreitet. Dadurch besitzt das Gewebe im Schnittbereich oder in der Zangenmitte ausreichend viel Feuchtigkeit und ist ausreichend leitfähig, um mittels des zweiten HF-Stroms schnell und effizient durchtrennt zu werden.

Die erste und die zweite Branche können jeweils ein Maulteil zum Fassen des Organs umfassen, wobei die Maulteile derart ausgebildet sind, dass sie sich in einem geschlossenen Zustand der Zange in einem Kontaktbereich kontaktieren. Vorzugsweise ist dieser Kontaktbereich mit einem oder mehreren Isolatoren versehen, so dass auch im geschlossenen Zustand der Zange die Koagulationselektroden von der Neutralelektrode elektrisch isoliert sind. Somit können Kurzschlüsse vermieden werden, die sich schädlich auf den verwendeten HF-Generator auswirken könnten.

Weitere vorteilhafte Ausführungsformen ergeben sich anhand der Unteransprüche.

Nachfolgend wird die Erfindung, welche in Anspruch 1 definiert ist, mittels mehrerer Ausführungsbeispiele beschrieben, die anhand von Abbildungen näher erläutert werden. Hierbei zeigen:
- Fig. 1 eine elektrochirurgische Zange;
- Fig. 2 ein erstes Ausführungsbeispiel der Maulteile der elektrochirurgischen Zange aus Fig. 1;
- Fig. 3 bis 6 weitere Ausführungsformen von nicht erfindungsgemäßen Maulteilen.

In der nachfolgenden Beschreibung werden für gleiche und gleich wirkende Teile dieselben Bezugsziffern verwendet.

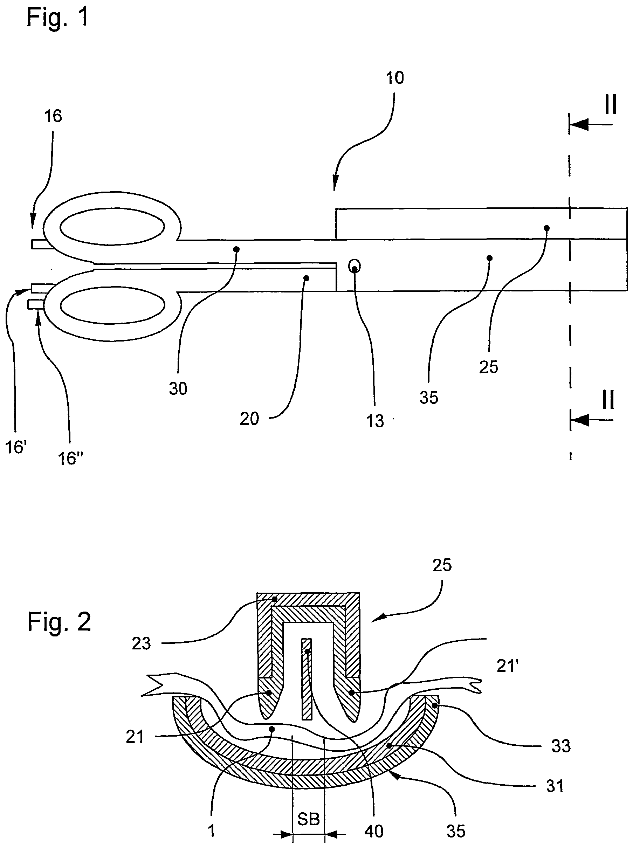

Fig. 1 zeigt schematisch dargestellt eine elektrochirurgische Zange 10, die aus einer ersten Branche 20 und einer zweiten Branche 30 besteht. Die einzelnen Branchen 20, 30 sind drehbeweglich über ein Drehgelenk 13 miteinander verbunden. Am proximalen Ende der Branchen 20, 30 befinden sich jeweils Handgriffe zum Bedienen der Zange 10. Die proximalen Enden weisen HF-Anschlüsse 16, 16', 16" auf, um das elektrochirurgische Instrument, also die Zange 10, mit einem HF-Generator zu verbinden. Das distale Ende der elektrochirurgischen Zange 10 weist ein erstes Maulteil 25 (Teil der ersten Branche 20) und ein zweites Maulteil 35 (Teil der zweiten Branche 30) auf.

Die Fig. 2 zeigt einen Schnitt durch diese Maulteile 25, 35. In einem geschlossenen Zustand der Zange 10 umgreift das zweite Maulteil 35 das erste Maulteil 25 zumindest abschnittsweise. In diesem geschlossenen Zustand lässt sich ein Hohlorgan, beispielsweise ein Gefäß 1, zwischen dem ersten Maulteil 25 und dem zweiten Maulteil 35 fixieren. In dem in Fig. 2 gezeigten Ausführungsbeispiel ist das zweite Maulteil 35 im Querschnitt als abgerundetes U-Profil ausgebildet. Es hat einen Rahmen 33, der aus einem elektrisch nichtleitfähigen Material besteht. Die gesamte Innenfläche des Rahmens 33 des zweiten Maulteils 35 ist mit einer Neutralelektrode 31 ausgekleidet, die zur Applikation eines HF-Stroms ausgebildet ist. Das erste Maulteil 25 ist als umgedrehtes U-Profil ausgebildet und hat ebenfalls einen elektrisch nichtleitfähigen Rahmen 23, dessen Innenfläche mit einem elektrisch leitfähigen Material ausgekleidet ist. Das elektrisch leitfähige Material überragt den Rahmen 23 in Richtung auf das zweite Maulteil 35 zu. Diese vorstehenden Abschnitte bilden eine erste und zweite Koagulationselektrode 21, 21' aus. Im geschlossenen Zustand der Zange 10 wird das Gefäß 1 zwischen der ersten Koagulationselektrode 21 und der Neutralelektrode 31 und zwischen der zweiten Koagulationselektrode 21' und der Neutralelektrode 31 eingeklemmt. Für das Verschließen des Gefäßes 1 kann in einer Koagulationsphase ein erster HF-Strom appliziert werden. Die entsprechende HF-Spannung liegt zwischen den Koagulationselektroden 21, 21' und der Neutralelektrode 31 an. Um einen Potentialunterschied zwischen den Koagulationselektroden 21, 21' zu vermeiden, sind diese elektrisch miteinander verbunden. Die beiden Koagulationselektroden 21, 21' sind über den HF-Anschluss 16' gemeinsam an den HF-Generator angeschlossen.

Die Koagulationselektroden 21, 21' sind im Querschnitt derart voneinander beabstandet, dass sich mittig zwischen diesen ein Aufnahmebereich bzw. eine Aussparung ergibt. Auch im geschlossenen Zustand der Zange 10 wird das Gefäß 1 unterhalb der Aussparung nicht von dem ersten Maulteil 25 kontaktiert. In diesem Bereich kann ein elektrisches Messer 40 entlang der Längsrichtung des ersten Maulteils 25 geführt werden, um nach der Koagulationsphase das Gefäß 1 zu durchtrennen. Hierfür wird eine HF-Spannung zwischen dem elektrischen Messer 40 und der Neutralelektrode 31 angelegt. Der applizierte zweite HF-Strom durchtrennt das Gefäß 1 in einem Schnittbereich SB (Schneidphase).

Obwohl in dem vorausgehenden Ausführungsbeispiel der Koagualations-Schneidvorgang als sequenzieller Prozess beschrieben wurde, ist es möglich, die Koagulations- und die Schneidphase gleichzeitig oder zeitlich zumindest teilweise überlappend auszuführen.

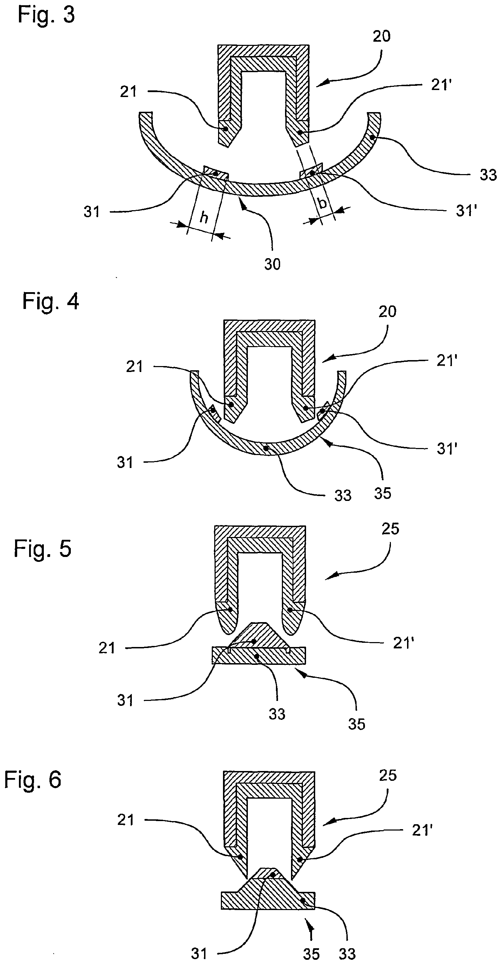

Die Fig. 3 zeigt ein weiteres Ausführungsbeispiel der erfindungsgemäßen Zange 10, wobei die Maulteile 25, 35 ebenfalls als U-Profile bzw. als umgedrehtes U-Profil ausgebildet sind. Die Enden der Koagulationselektroden 21, 21' sind jedoch gegenüber denen der Fig. 2 (dort konvex) abgeflacht ausgebildet. Des Weiteren hat das zweite Maulteil 35 nicht nur eine großflächige Neutralelektrode 31, sondern zwei räumlich voneinander getrennt angeordnete Neutralelektroden 31, 31'. Diese abgeflachten Enden des ersten Maulteils 25 verlaufen im Wesentlichen parallel zu den beiden Neutralelektroden 31, 31', die am Rahmen 33 der zweiten Branche 30 angeordnet sind. Die Koagulationselektroden 21, 21' haben eine definierte Koagulationselektrodenbreite b, die im geschlossenen Zustand der Zange 10 auf der ersten Neutralelektrode 31 bzw. auf der zweiten Neutralelektrode 31' mit der Neutralelektrodenbreite h aufliegen. Erfindungsgemäß sollen die Koagulationselektrodenbreiten b und die Neutralelektrodenbreiten h relativ gering ausgebildet sein, so dass ein hoher HF-Stromeintrag in einen räumlich beschränkten Bereich des Gefäßes 1 erfolgt. Ein Austrocknen des Gefäßes 1 im Schnittbereich SB kann somit vermieden werden.

Die Koagulationselektroden 21, 21' gemäß dem Ausführungsbeispiel der Fig. 3 sind gegenüber der Horizontalen leicht geneigt. Somit ergibt sich beim Schließen der Zange 10, wobei das Gefäß 1 die Koagulationselektroden 21, 21' nur teilweise kontaktiert, eine wesentlich geringere Wirkfläche. Vorzugsweise sind wie in Fig. 3 dargestellt, auch die Neutralelektroden 31, 31' entsprechend geneigt.

Bei dem Ausführungsbeispiel gemäß Fig. 4 sind die Neutralelektroden 31, 31' nicht unterhalb, sondern neben den Koagulationselektroden 21, 21' angeordnet. Im geschlossenen Zustand kontaktieren die Koagulationselektroden 21, 21' den Rahmen 33 des zweiten Maulteils 35 unmittelbar. Es besteht kein Kontaktschluss zwischen den Neutralelektroden 31, 31' und den Koagulationselektroden 21, 21'. Die Neutralelektroden 31, 31' sind derart am Rahmen 33 des zweiten Maulteils 35 angeordnet, dass die Koagulationselektroden 21, 21' im geschlossenen Zustand der Zange 10 neben diesen zu liegen kommt. Sobald Gewebe eingeklemmt ist, ergibt sich ein kürzester Strompfad von den Seitenbereichen der Koagulationselektroden 21, 21' zu den Neutralelektroden 31, 31'. Während sich der Schnittbereich SB zwischen den Koagulationselektroden 21, 21' befindet, sind die Neutralelektroden wechselseitig außerhalb des von dem zweiten Maulteil 35 überdeckten Bereichs angeordnet. Somit ist der Schnittbereich SB durch das zweite Maulteil 35, insbesondere durch die daran angeordneten Koagulationselektroden 21, 21' elektrisch abgeschirmt. Ein Einwirken des ersten HF-Stroms auf den von dem zweiten Maulteil 35 umklammerten Schnittbereich SB ist äußerst unwahrscheinlich.

In einer weiteren Ausführungsform der Erfindung ist die Neutralelektrode 31 mittig zwischen den Koagulationselektroden 21, 21' angeordnet (vgl. Fig. 5). Die Neutralelektrode 31 bildet einen Steg aus, der sich entlang der Längsrichtung des zweiten Maulteils 35 erstreckt und im geschlossenen Zustand von dem ersten Maulteil 25 umfasst wird.

Im geschlossenen Zustand der Zange 10 ergeben sich auch in dem Ausführungsbeispiel gemäß Fig. 5 Wirkflächen der Neutralelektrode 31, die seitlich angeordnet sind. Somit wird ein möglicherweise auf dem Steg aufliegendes Gefäß 1 im Schnittbereich SB durch den ersten HF-Strom nicht geschädigt, da wesentlich kürzere Strompfade, die horizontal verlaufen, bereitstehen. Der Steg des Ausführungsbeispiels gemäß der Fig. 5 ist flächig auf einem im Wesentlichen ebenen Rahmen 33 des zweiten Maulteils 35 angeordnet.

Demgegenüber bildet im Ausführungsbeispiel gemäß Fig. 6 der Rahmen 33 bereits einen Teil der sich entlang der Längsrichtung des zweiten Maulteils 35 erstreckenden Stegs aus. Nur ein oberer Abschnitt des Stegs ist mit der Neutralelektrode 31 versehen. Die Neutralelektrode 31 ist also derart angeordnet, dass im geschlossenen Zustand der Zange 10 kein unmittelbarer Kontakt zwischen den Koagulationselektroden 21, 21' und der Neutralelektrode 31 vorliegt. Der Kontakt kann lediglich über das Gewebe hergestellt werden. Somit wird ein Kurzschluss zwischen den Elektroden 21, 21', 31 vermieden. Im geschlossenen Zustand liegt ein Teilabschnitt der Koagulationselektroden 21, 21' auf dem Rahmen 33 des zweiten Maulteils 35 auf. Somit ist der gesamte Kontaktbereich auf der Seite des zweiten Maulteils 35 als elektrischer Isolator ausgebildet.

### Bezugszeichenliste

- 1: Gefäß
- 10: elektrochirurgische Zange
- 13: Drehgelenk
- 16, 16', 16": HF-Anschluss
- 20: erste Branche
- 21, 21': Koagulationselektrode
- 23: Rahmen
- 25: erstes Maulteil
- 30: zweite Branche
- 31, 31': Neutralelektrode
- 33: Rahmen
- 35: zweites Maulteil
- 40: elektrisches Messer
- b: Koagulationselektrodenbreite
- h: Neutralelektrodenbreite
- SB: Schnittbereich

## Patentansprüche

1. Elektrochirurgische Zange mit einer ersten Branche (20) und einer zweiten Branche (30) zum Fassen eines Hohlorgans (1),
umfassend:
- mindestens zwei Neutralelektroden (31, 31') an der zweiten Branche (30), wobei ein Teil der zweiten Branche (30) als U-Profil ausgebildet ist, wobei die beiden Neutralelektroden (31, 31') räumlich voneinander getrennt angeordnet sind;
- mindestens eine erste Koagulationselektrode (21) und eine zweite Koagulationselektrode (21'), die an der ersten Branche (20) zum Applizieren eines ersten HF-Stroms mittels der Koagulationselektroden (21, 21') und der beiden Neutralelektroden (31) angeordnet sind;
- mindestens eine Schneidvorrichtung (40), die zwischen den Koagulationselektroden (21, 21') angeordnet ist, um das Hohlorgan (1) in einem Schnittbereich (SB) zu durchtrennen,
wobei
die elektrochirurgische Zange mindestens eine Aussparung zur Aufnahme des Schnittbereichs (SB) beim Fassen des Hohlorgans (1) aufweist, und die Koagulationselektroden (21, 21') im Querschnitt derart voneinander beabstandet sind, dass sich mittig zwischen den Koagulationselektroden (21, 21') die Aussparung ergibt, und wobei die Schneidvorrichtung (40) mindestens eine Schneidelektrode zum Applizieren eines zweiten HF-Stroms mittels der Schneidelektrode und der beiden Neutralelektroden (31, 31') umfasst, **dadurch gekennzeichnet, dass** die Koagulationselektroden (21, 21') derart voneinander beabstandet angeordnet sind, dass der erste HF-Strom bei dessen Applikation den Schnittbereich (SB) nicht oder nur in geringem Maße durchfließt, sodass im Schnittbereich keine Koagulation vorkommt, wobei ein Teil der ersten Branche als U-Profil ausgebildet ist und abgeflachte Enden aufweist, die im Wesentlichen parallel zu den beiden Neutralelektroden (31, 31') verlaufen, wobei die Koagulationselektroden (21, 21') eine geringe Koagulationselektrodenbreite (b) von kleiner gleich 2 Millimetern haben.

2. Elektrochirurgische Zange nach Anspruch 1,
**dadurch gekennzeichnet, dass**
die Koagulationselektroden (21, 21') derart ausgebildet und positioniert sind, dass der erste HF-Strom bei dessen Applikation den Schnittbereich (SB) nicht durchfließt, wobei durch den zweiten HF-Strom ein Schnitt durchgeführt wird.

3. Elektrochirurgische Zange nach Anspruch 1 oder 2,
**dadurch gekennzeichnet, dass**
die Koagulationselektroden (21, 21') gegenüber der Horizontalen leicht geneigt sind.

4. Elektrochirurgische Zange nach einem der vorhergehenden Ansprüche,
**dadurch gekennzeichnet, dass**
das U-Profil der zweiten Branche (30) als gerundetes U-Profil ausgebildet ist

5. Elektrochirurgische Zange nach einem der vorhergehenden Ansprüche,
**dadurch gekennzeichnet, dass**
die Neutralelektroden (31, 31') geneigt sind.

6. Elektrochirurgische Zange nach einem der vorhergehenden Ansprüche,
**dadurch gekennzeichnet, dass**
die erste und die zweite Neutralelektrode (31, 31') jeweils eine Ebene aufspannen und derart angeordnet sind, dass die Ebenen sich unter einem Winkel, insbesondere einem spitzen Winkel, schneiden.

7. Elektrochirurgische Zange nach einem der vorhergehenden Ansprüche,
**dadurch gekennzeichnet, dass**
die Koagulationselektroden (21, 21') jeweils eine Ebene aufspannen und derart angeordnet sind, dass die Ebenen sich unter einem Winkel, insbesondere einem spitzen Winkel, schneiden.

8. Elektrochirurgische Zange nach einem der vorhergehenden Ansprüche,
**dadurch gekennzeichnet, dass**
die Koagulationselektroden (21, 21') eine geringe Koagulationselektrodenbreite (b), insbesondere von kleiner gleich 3 Millimetern oder von kleiner gleich 2 Millimetern haben.

9. Elektrochirurgische Zange nach einem der vorhergehenden Ansprüche,
**dadurch gekennzeichnet, dass**
die erste und die zweite Branche (20, 30) Maulteile umfassen, die derart ausgebildet sind, dass sie sich in einem geschlossenen Zustand der Zange in einem Kontaktbereich kontaktieren, wobei der Kontaktbereich einen Isolator zur elektrischen Isolation der Koagulationselektroden (21, 21') gegenüber der mindestens eine Neutralelektrode (31, 31') im geschlossenen Zustand umfasst.

## Claims

1. Electrosurgical forceps with a first branch (20) and a second branch (30) for gripping a hollow organ (1), comprising
- at least two neutral electrodes (31, 31') on the second branch (30), wherein a part of the second branch (30) is designed as a U-profile, wherein the two neutral electrodes (31, 31') are arranged spatially separated from one another;
- at least one first coagulation electrode (21) and a second coagulation electrode (21'), which are arranged on the first branch (20) for applying a first RF current by means of the coagulation electrodes (21, 21') and the two neutral electrodes (31);
- at least one cutting device (40), arranged between the coagulation electrodes (21, 21') for severing the hollow organ (1) in a cutting region (SB),
wherein
the electrosurgical forceps has at least one recess for holding the cutting region (SB) when gripping the hollow organ (1) and the coagulation electrodes (21, 21') are spaced apart from one another in the cross section such that this results in the recess centrally between the coagulation electrodes (21, 21'), and wherein the cutting device (40) comprises at least one cutting electrode for applying a second RF current by means of the cutting electrode and the two neutral electrodes (31, 31'), **characterized in that** the coagulation electrodes (21, 21') are arranged at a distance from one another such that the first RF current does not flow, or hardly flows, through the cutting region (SB) when it is applied, such that there is no coagulation in the cutting region, wherein a part of the first branch is designed as a U-profile and has flattened ends that run substantially parallel to the two neutral electrodes (31, 31'), wherein the coagulation electrons (21, 21') have a small coagulation electrode width (b) of less than or equal to 2 millimeters.

2. Electrosurgical forceps according to Claim 1,
**characterized in that**
the coagulation electrodes (21, 21') are embodied and positioned in such a way that the first RF current does not flow through the cutting region (SB) when it is applied, wherein the second RF current carries out a cut.

3. Electrosurgical forceps according to Claim 1 or 2,
**characterized in that**
the coagulation electrodes (21, 21') are slightly tilted with respect to the horizontal.

4. Electrosurgical forceps according to one of the preceding claims,
**characterized in that**
the U-profile of the second branch (30) is embodied as a rounded U-profile.

5. Electrosurgical forceps according to one of the preceding claims,
**characterized in that**
the neutral electrodes (31, 31') are tilted.

6. Electrosurgical forceps according to one of the preceding claims,
**characterized in that**
the first and the second neutral electrode (31, 31') respectively span a plane and are arranged such that the planes intersect at an angle, more particularly at an acute angle.

7. Electrosurgical forceps according to one of the preceding claims,
**characterized in that**
the coagulation electrodes (21, 21') respectively span a plane and are arranged such that the planes intersect at an angle, more particularly at an acute angle.

8. Electrosurgical forceps according to one of the preceding claims,
**characterized in that**
the coagulation electrodes (21, 21') have a small coagulation electrode width (b), more particularly of less than or equal to 3 millimeters or of less than or equal to 2 millimeters.

9. Electrosurgical forceps according to one of the preceding claims,
**characterized in that**
the first and the second branch (20, 30) comprise jaw parts that are designed such that they contact one another in a contact region when the forceps is in a closed state, the contact region comprising an insulator for electric insulation of the coagulation electrodes (21, 21') from the at least one neutral electrode (31, 31') in the closed state.

## Revendications

1. Pince électrochirurgicale, comprenant une première branche (20) et une deuxième branche (30) destinées à saisir un organe creux (1),
comportant :
- au moins deux électrodes neutres (31, 31') sur la deuxième branche (30), une partie de la deuxième branche (30) étant réalisée sous la forme d'un profilé en U, les deux électrodes neutres (31, 31') étant montées séparées l'une de l'autre dans l'espace ;
- au moins une première électrode de coagulation (21) et une deuxième électrode de coagulation (21'), lesquelles sont disposées sur la première branche (20) pour appliquer un premier courant HF au moyen des électrodes de coagulation (21, 21') et des deux électrodes neutres (31) ;
- au moins un dispositif de coupe (40) qui est disposé entre les électrodes de coagulation (21, 21') afin de sectionner l'organe creux (1) dans une zone de coupe (SB),
la pince électrochirurgicale possédant au moins une cavité servant à accueillir la zone de coupe (SB) lors de la saisie de l'organe creux (1), et les électrodes de coagulation (21, 21') étant espacées l'une de l'autre en section transversale de telle sorte que la cavité est produite au centre entre les électrodes de coagulation (21, 21'), et le dispositif de coupe (40) comportant au moins une électrode de coupe destinée à appliquer un deuxième courant HF au moyen de l'électrode de coupe et des deux électrodes neutres (31, 31'),
**caractérisé en ce que**
les électrodes de coagulation (21, 21') sont disposées espacées l'une de l'autre de telle sorte que le premier courant HF, lors de son application, ne traverse pas la zone de coupe (SB), ou seulement dans une faible mesure, de sorte qu'il ne se produit pas de coagulation dans la zone de coupe, une partie de la première branche étant réalisée sous la forme d'un profilé en U et présente des extrémités aplaties qui s'étendent sensiblement en parallèle des deux électrodes neutres (31, 31'), les électrodes de coagulation (21, 21') ayant une largeur d'électrode de coagulation (b) inférieure ou égale à 2 millimètres.

2. Pince électrochirurgicale selon la revendication 1, **caractérisée en ce que** les électrodes de coagulation (21, 21') sont configurées et positionnées de telle sorte que le premier courant HF ne traverse pas la zone de coupe (SB) lors de son application, une coupe étant effectuée par le deuxième courant HF.

3. Pince électrochirurgicale selon la revendication 1 ou 2, **caractérisée en ce que** les électrodes de coagulation (21, 21') sont légèrement inclinées par rapport à l'horizontale.

4. Pince électrochirurgicale selon l'une des revendications précédentes, **caractérisée en ce que** le profilé en U de la deuxième branche (30) est réalisé sous la forme d'un profilé en U arrondi.

5. Pince électrochirurgicale selon l'une des revendications précédentes, **caractérisée en ce que** les électrodes neutres (31, 31') sont inclinées.

6. Pince électrochirurgicale selon l'une des revendications précédentes, **caractérisée en ce que** les première et deuxième électrodes neutres (31, 31') couvrent respectivement un plan et sont disposées de telle sorte que les plans se croisent sous un angle donné, notamment un angle aigu.

7. Pince électrochirurgicale selon l'une des revendications précédentes, **caractérisée en ce que** les électrodes de coagulation (21, 21') couvrent respectivement un plan et sont disposées de telle sorte que les plans se croisent sous un angle donné, notamment un angle aigu.

8. Pince électrochirurgicale selon l'une des revendications précédentes, **caractérisée en ce que** les électrodes de coagulation (21, 21') possèdent une faible largeur d'électrode de coagulation (b), notamment inférieure ou égale à 3 millimètres ou inférieure ou égale à 2 millimètres.

9. Pince électrochirurgicale selon l'une des revendications précédentes, **caractérisée en ce que** les première et deuxième branche (20, 30) comprennent des parties de mâchoire qui sont configurées de telle sorte qu'elles entrent en contact l'une avec l'autre dans une zone de contact dans un état fermé de la pince, la zone de contact comprenant un isolateur servant à l'isolation électrique des électrodes de coagulation (21, 21') par rapport à l'au moins une électrode neutre (31, 31') dans l'état fermé.
